(19) Europäisches Patentamt
European Patent Office
Office européen des brevets

(11) Publication number: **0 423 617 A1**

## (12) EUROPEAN PATENT APPLICATION

(21) Application number: **90119494.4**

(22) Date of filing: **11.10.90**

(51) Int. Cl.[5]: **C07H 19/167**, C07H 19/173, C07H 19/052, C07H 19/056, A61K 31/70

(30) Priority: **11.10.89 US 419990**

(43) Date of publication of application: **24.04.91 Bulletin 91/17**

(84) Designated Contracting States: **AT BE CH DE DK ES FR GB GR IT LI LU NL SE**

(71) Applicant: **MERRELL DOW PHARMACEUTICALS INC.**
**2110 East Galbraith Road**
**Cincinnati Ohio 45215(US)**

(72) Inventor: **Jarvi, Esa T.**
**3953 St.Johns Terrace**
**Cincinnati, Ohio 45236(US)**
Inventor: **Prakash, Nellikunja L.**
**10871 Wengate Lane**
**Cincinnati, Ohio 45241(US)**
Inventor: **McCarthy, James R. L.**
**6448 Foxview Place**
**West Chester, Ohio 45069(US)**

(74) Representative: **Vossius & Partner**
**Siebertstrasse 4 P.O. Box 86 07 67**
**W-8000 München 86(DE)**

# (54) Inosine/Guanosine derivatives as antineoplastic agents.

(57) This invention relates to certain inosine and guanosine derivatives of formula (1) which are inhibitors of purine nucleoside phosphorylases and are useful in the treatment of patients suffering from certain neoplastic diseases including leukemia, melanomas, carcinomas, sarcomas and mixed neoplasias

1 ...na of the form a (1)

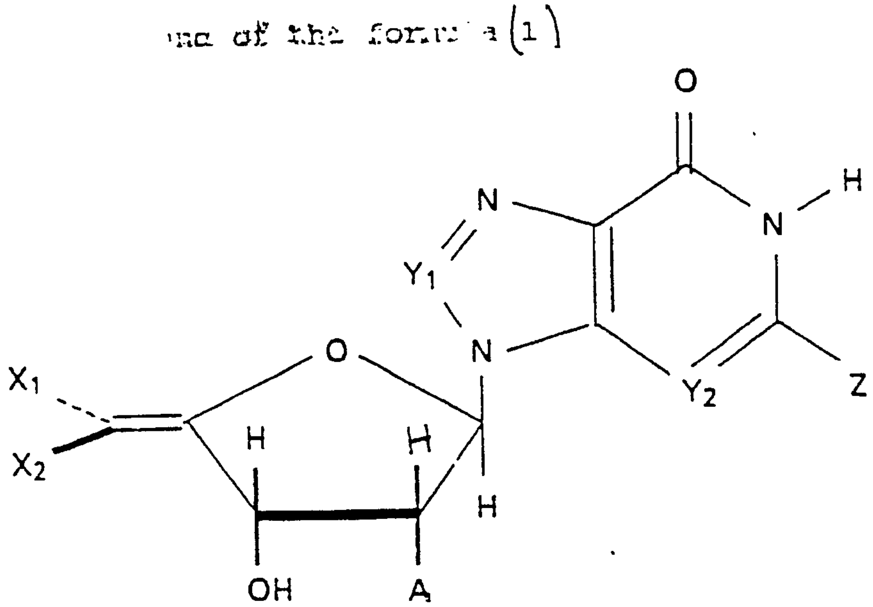

(1)

EP 0 423 617 A1

wherein
$X_1$ and $X_2$ are each independently hydrogen or halogen with the proviso that at least one of $X_1$ and $X_2$ is a halogen atom,
A is hydrogen or hydroxy,
$Y_1$ is nitrogen, a CH group, a CCl group, a CBr group or a $CNH_2$ group, and
Z is hydrogen, halogen, or $NH_2$.

## INOSINE/GUANOSINE DERIVATIVES AS ANTINEOPLASTIC AGENTS

The present invention relates to certain inosine and guanosine derivatives which are useful as inhibitors of purine nucleoside phosphorylases. The compounds of the present invention are useful in the treatment of patients suffering from certain neoplastic diseases including leukemia, melanomas, carcinomas, sarcomas and mixed neoplasias.

Purine nucleoside phosphorylases (PNP) are enzymes present in mammalian cells and represent the primary mechanism by which animal tissues degrade the ribonucleosides and the deoxyribonucleosides of guanine and hypoxanthine (i.e., guanosine/deoxyguanosine and inosine/deoxyinosine) to the free bases and the respective pentose-1-phosphates. This reaction catalyzed by PNP is readily reversible.

Various lines of evidence have led to the conclusion that PNP inhibitors have therapeutic activity. For example, it has been suggested by Parks et al. ["Purine Nucleoside Phosphorylase and 5′-Methylthioadenosine Phosphorylase: Targets of Chemotherapy" in MOLECULAR ACTIONS AND TARGETS FOR CANCER CHEMOTHERAPEUTIC AGENTS, 1981, Academic Press Inc.] that "[S]ince children with inheritable PNP deficiencies display normal humoral but defective cellular immunity, and the activity of PNP in T lymphocytes is higher than in B lymphocytes, one may predict that inhibition of PNP will have specific effects on T lymphocytes and cell-mediated immunity" (*Id.* pages 231-32). It has also been found that excessive amounts of deoxyguanosinetriphosphate (dGTP) accumulates in the tissues of PNP-deficient individuals [*Id.* page 232]. The nucleoside dGTP is a potent feed-back inhibitor of ribonucleotide reductase, an enzyme essential for the synthesis of deoxynucleotides during DNA synthesis.

Therefore, PNP inhibition would reasonably be expected to result in inhibition of proliferation of rapidly dividing cells. PNP inhibitors would therefore be useful as as antineoplastic agents and PNP inhibitors would provide effective therapy in the treatment of various neoplastic diseases.

The present invention relates to certain novel inosine/guanosine derivatives of the formula (1)

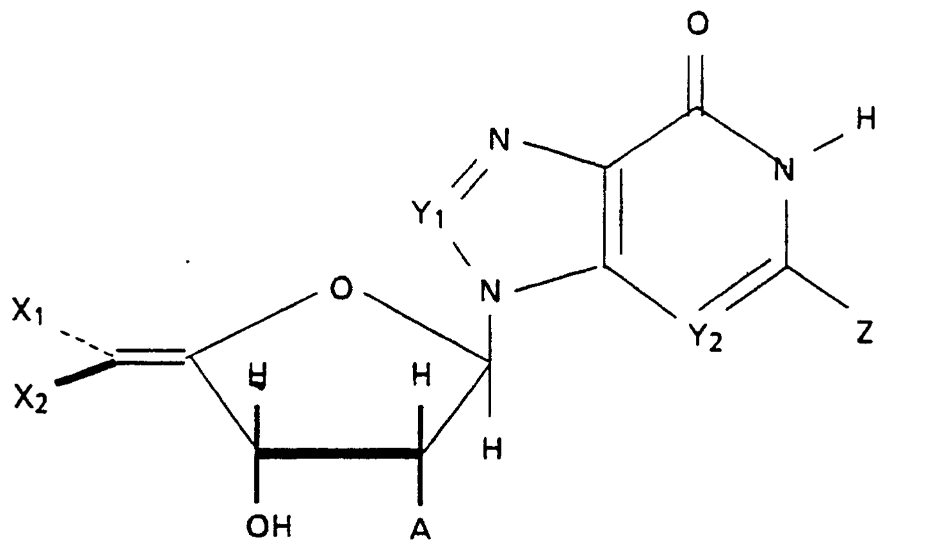

(1)

wherein

$X_1$ and $X_2$ are each independently hydrogen or halogen with the proviso that at least one of $X_1$ and $X_2$ is a halogen atom,

A is hydrogen or hydroxy,

$Y_2$ is nitrogen, a CH group, a CCl group, a CBr group or a $CNH_2$ group,

$Y_2$ is nitrogen or a CH group, and

Z is hydrogen, halogen, or $NH_2$.

These compounds are inhibitors of purine nucleoside phosphorylase. The present invention provides a method for treating a patient suffering from a neoplastic disease comprising administering to said patient a therapeutically effective antineoplastic amount of a compound of formula (1).

The present invention also provides a method for inhibiting a purine nucleoside phosphorylase in a patient in need thereof comprising administering to said patient an effective purine nucleoside phosphorylase inhibitory amount of a compound of formula (1).

As used herein, the terms "halogen" or "halo-" refer to a monovalent fluorine, chlorine, bromine or iodine atom. Of course, it will be appreciated by those skilled in the art that the inosine/guanosine derivatives of the present invention can exist in the keto or enol form. For purposes of convenience, the structures of these inosine/guanosine derivatives are presented in the keto form only. The present invention is not limited to any one form of the inosine/guanosine derivatives and both the keto and enol forms are included within the scope of the invention. It will further be appreciated that the inosine/guanosine derivatives of the present invention can exist in various stereoisomeric forms. The present invention is not limited to any particular stereoisomeric forms and includes all such stereoisomers individually and as racemic mixtures.

The inosine/guanosine derivatives of the present invention can be prepared by utilizing procedures and techniques well known and appreciated by one of ordinary skill in the art.

A general synthetic procedure for making inosine/guanosine derivatives of the formula (1), wherein Z is other than $NH_2$, is set forth in Scheme A. In this scheme the compounds of formula (1) are made from the corresponding 4′-vinylhalo-adenosine derivatives. The term $Z_1$ refers to hydrogen or halogen and all other substituents, unless otherwise indicated, are as previously defined.

Scheme A

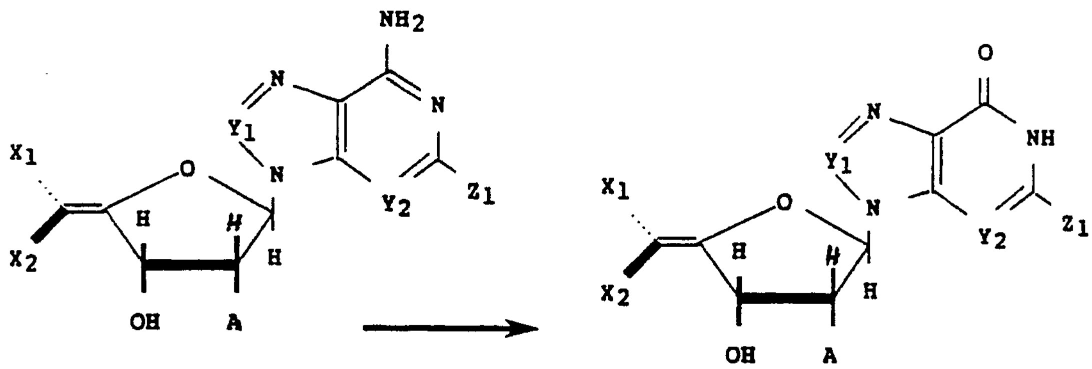

(2) (3)

In this reaction, compounds of the formula (1) are made by subjecting a corresponding 4′-vinylhalo-adenosine derivative to an acidic oxidative deamination.

In Scheme A, the 4′-vinylhalo-adenosine derivative (2) is subjected to an oxidative deamination by means of an acidic oxidative agent to yield the corresponding 4′-vinylhalo-inosine/guanosine derivative (3). The preferred acidic oxidative agent for this reaction is nitrous acid. Where nitrous acid is utilized, the 4′-vinylhalo-adenosine derivative (2) can be dissolved in an organic acid such as acetic acid and sodium nitrite can be added to form the nitrous acid *in situ* The reaction can generally be carried out at ambient temperature and is completed within several hours.

The following examples present typical syntheses as described by Scheme A. These examples are understood to be illustrative only and are not intended to limit the scope of the present invention in any way. As used herein the following abbreviated terms have the following meanings: mg refers to milligrams, mmol refers to millimoles, mL refers to milliliters, v refers to volume.

EXAMPLE 1

(Z)-5′-Fluoro-4′,5′-didehydro-5′-deoxyinosine

Dissolve (Z)-5′-fluoro-4′,5′-didehydro-5′-deoxyadenosine (267 mg, 1 mmol) in glacial acetic acid (10

mL) with stirring. To this reaction mixture add a solution of sodium nitrite (276 mg, 4 mmol) in water (2 mL). Stir the mixture for 5 hours at ambient temperature. Evaporate the solvent to dryness *in vacuo* and triturate the resulting solid with boiling acetone. Evaporate the acetone extracts to dryness. Recrystallize the title compound from ethanol/water.

## EXAMPLE 2

### (Z)-5′-Chloro-4′,5′-didehydro-5′-deoxyinosine

Dissolve (Z)-5′-chloro-4′,5′-didehydro-5′-deoxyadenosine (284 mg, 1 mmol) in glacial acetic acid (10 mL) with stirring. To this reaction mixture add a solution of sodium nitrite (276 mg, 4 mmol) in water (2 mL). Stir the mixture for 5 hours at ambient temperature. Evaporate the solvent to dryness *in vacuo* and triturate the resulting solid with boiling acetone. Evaporate the acetone extracts to dryness. Recrystallize the title compound from ethanol/water.

The 4′-vinylhalo-adenosine derivatives (2) which are useful as starting materials in the reaction described in Scheme A, can be prepared by utilizing procedures and techniques which are well known and appreciated by those of ordinary skill in the art.

For example, the 4′-vinylhalo-adenosine derivatives, wherein one of $X_1$ and $X_2$ is hydrogen, can be prepared by a general synthetic procedure as set forth in Scheme B wherein the term "halogen" or "$X_{Hal}$" refers to a fluorine, chlorine, bromine, or iodine atom and the term "nitrogen" refers to a trivalent nitrogen atom attached to two radicals and all other substituents, unless otherwise indicated, are as previously defined.

SCHEME B

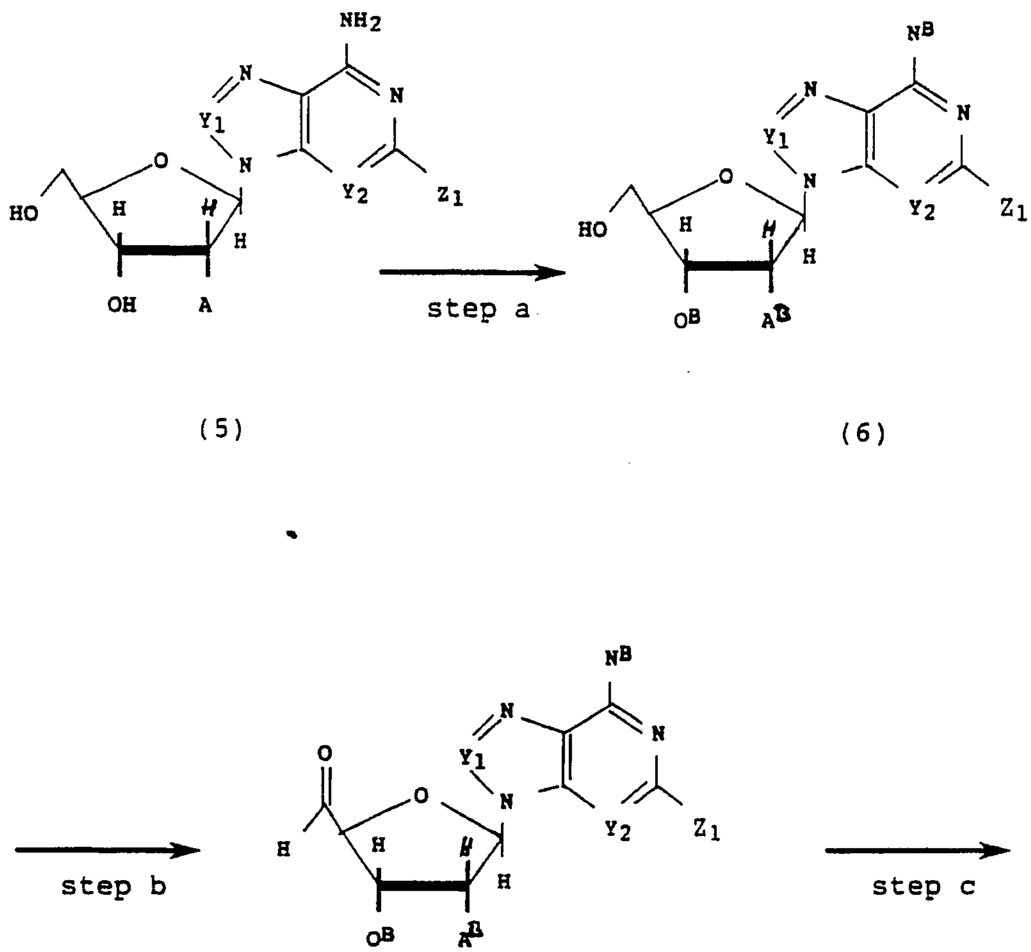


(5)

(6)

(7)

Scheme B (cont'd)

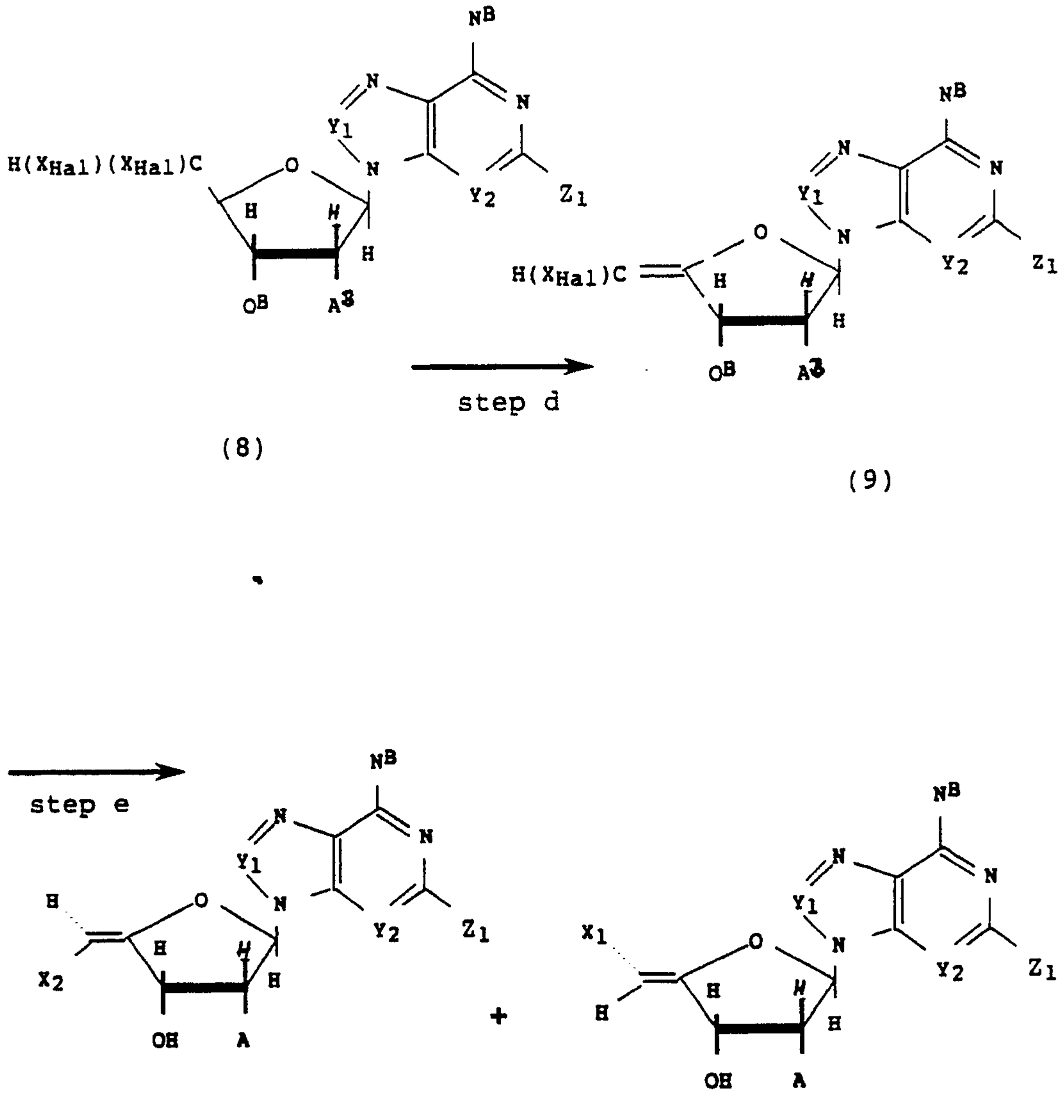


(8) (9)

(10) (11)

Scheme B (cont'd)

(10)

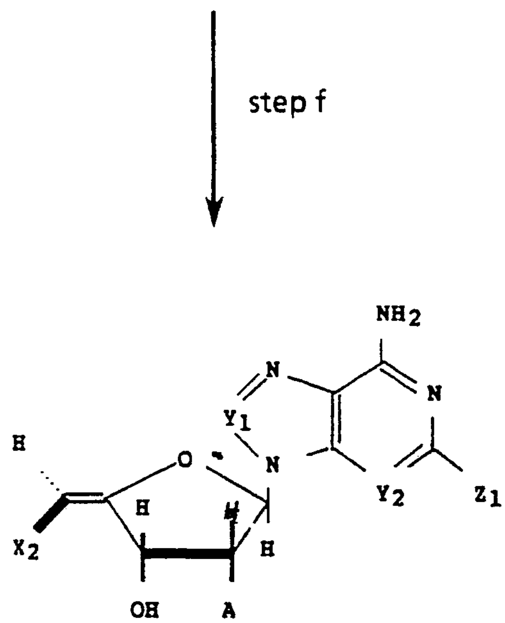


(11)

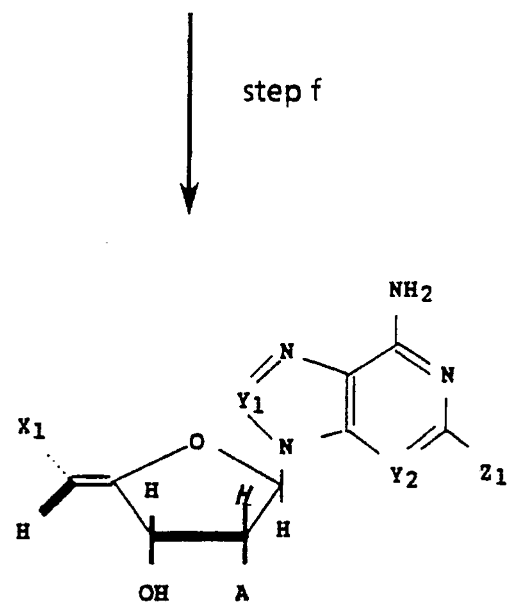


(12)

(13)

Basically, in step a, reactive hydroxy or amino groups other than the 5′-hydroxy group are blocked with standard blocking agents well known in the art. These blocking groups can be conventional amino protecting groups for the 6-amino, and conventional hydroxy protecting groups for the 3′-hydroxy and for A (wherein A is OH). $O^B$, $A^B$, $A^B$and $N^B$ in Scheme B represent the 3′-hydroxy, A and the 6-amino groups as herein defined blocked with a blocking group where appropriate.

The selection and utilization of particular blocking groups are well known to one of ordinary skill in the art. In general, blocking groups should be selected which adequately protect the amino or hydroxy groups in question during subsequent synthetic steps and which are readily removable under conditions which will not cause degradation of the desired product.

Examples of suitable hydroxy protecting groups are $C_1$-$C_6$ alkyl, tetrahydropyranyl, methoxymethyl, methoxyethoxymethyl, t-butyl, benzoyl, and triphenylmethyl. The term $C_1$-$C_6$ alkyl refers to a saturated hydrocarbyl radical of one to six carbon atoms of straight, branched, or cyclic configuration. The preferred blocking group for the 3′-hydroxy and for A (wherein A is hydroxy) is 2′,3′-0-isopropylidene. The preferred blocking group for the 3′-hydroxy (wherein A is not hydroxy) is benzoyl. The 2′,3′-0-isopropylidene derivative can be formed by reacting the unblocked compound with acetone. The benzoylated derivative can be formed by reacting the unblocked compound with benzoyl chloride in the presence of pyridine.

Examples of suitable amino protecting groups are benzoyl, formyl, acetyl, trifluoroacetyl, phthalyl, tosyl, benzenesulfonyl, benzyloxycarbonyl, substituted-benzyloxycarbonyl (e.g., p-chloro,p-bromo, p-nitro, p-methoxy, o-chloro, 2,4-dichloro, and 2,6-dichloro derivatives), t-butyloxycarbonyl (Boc), t-amyloxycarbonyl, isopropyloxycarbonyl, 2-(p-biphenyl)-isopropyloxycarbonyl, allyloxycarbonyl, cyclopentyloxycarbonyl, cyclohexyloxycarbonyl, adamantyloxycarbonyl, phenylthiocarbonyl, and triphenylmethyl. Preferred amino protected compounds include the di-benzoyl derivative, made by reacting the unblocked compound with benzoyl chloride, and then acetyl derivative, made by reacting the unblocked compound with acetic

anhydride. It is particularly preferred to protect the 6-amino group as the $N^6$,$N^6$-di-benzoyl derivative

In step b, the appropriately blocked 5′-hydroxy derivative (6) is oxidised to the corresponding aldehyde (7). The preferred oxidising reagent is dicyclohexylcarbodiimide, methyl phosphonic or dichloroacetic acid and dimethylsulfoxide

The aldehyde (7) can optionally be derivatized so as to improve the handling characteristics of the compound or to facilitate purification thereof by means of procedures and techniques well-known and appreciated in the art. For example, the 5′,5′-(N,N′-diphenylethylenediamino) derivative can be prepared by the method of Ranganathan et al. (J. Org. Chem., 39 , 290 (1974)].

In step c, the 5′,5′-di-halo derivative (8) is formed by reacting the corresponding aldehyde (7) with a diethylaminosulfur trihalide or similar halo-substituting reagent. Diethylaminosulfur trihalide is preferred.

In step d, the 5′-di-halo derivative (8) is dehydrohalogenated to form the unsaturated (i.e., "(H) ($X_{Hal}$)C") derivative (9). The preferred reagent to effect the dehydrohalogenation is potassium t-butoxide in the presence of dimethylsulfoxide.

In step e, the hydroxy protecting groups are removed according to conventional procedures and techniques well known and appreciated in the art. For example, a 2′,3′-0- isopropylidene blocking group can be removed by reacting (9) with aqueous trifluoroacetic acid. The (Z) and (E) isomers, i.e., (10) and (11), respectively, can conveniently be isolated at this stage of the synthesis by the utilization of conventional isolation techniques as are well known and appreciated in the art. Alternatively, the (Z) and (E) isomers can be isolated after deblocking the amino-protecting groups as described below for steps f and g.

In step f, the amino-protecting groups of the (Z) and (E) isomers, i.e., (10) and (11) respectively, are removed utilizing procedures and techniques well known and appreciated in the art. For example, the benzoyl amino blocking groups can be removed by an aminolysis reaction with ammonia.

Starting materials for use in the general synthetic procedure outlined in Scheme B are readily available to one of ordinary skill in the art. For example, certain starting materials for various compounds of formula (1) are listed in Table 1.

TABLE 1

| Examples of Starting Materials for Scheme B | | | | | |
|---|---|---|---|---|---|
| Compound of formula (1) wherein | | | | | |
| $A_1$ | $A_2$ | $Y_1$ | $Y_2$ | Z | Source of Starting Material |
| H | OH | CH | CH | H | J. Med. Chem. 25, 626(1982) |
| H | H | CH | N | H | 2′-Deoxyadenosine(commercially available) |

Additional starting materials can be prepared by the use of methods analogous to those described in Table 1 as well as other convential methods as are well known and appreciated in the art.

The following example presents a typical synthesis as described by Scheme B. This example is understood to be illustrative only and is not intended to limit the scope of the present invention in any way.

## EXAMPLE 3

(Z) and (E)-4′,5′-Didehydro-5′-deoxy-5′-fluoroadenosine

Step a: $N^6$-benzoyl-2′,3′-0-isopropylidene-adenosine.

Convert adenosine to its 2′,3′-acetonide followed by benzoylation to the $N^6$-benzoyl derivative according to the procedure of Smrt et al. [ Coll. Czech. Chem. Comm. 29 , 224 (1964)].

Step b: $N^6$,$N^6$-Bis benzoyl-5-deoxy-2′,3′-0-isopropylidene-5′-,5′-(N,N′-diphenylethylenediamino) adenosine.

Convert $N^6$-benzoyl-2′,3′-0-isopropylidene adenosine to $N^6$-benzoyl-5′-deoxy-2′,3′-0-isopropylidene-5′,5′-(N,N′-diphenylethylenediamino)adenosine according to the procedure of Ranganathan et al. [J. Org. Chem. 39 , 290 (1974)]. To 2.96 g of this product in 10 ml of pyridine, cooled in an ice bath, add 1.15 ml (9.9 mmol) of benzoyl chloride. Stir the mixture overnight at room temperature and pour into ice water. Extract the product into 100 ml of chloroform and dry with magnesium sulfate. Evaporate the solution on a rotary evaporator and add toluene. Repeat the evaporation *in vacuo*, and collect 4.07 g of a yellow foam. Percolate the product through a 40 mm X 10 cm flash silica gel column with 4% ethyl acetate/96% dichloromethane. Combine and evaporate the appropriate fractions and collect a yellow oil. Dissolve the oil in ethanol and evaporate three times to yield a solid. Triturate the solid with 50 ml of ethanol and filter. Dry the solid *in vacuo* to give 2.67 g of the title compound [mp 135-138 degrees Celsius (° C)].

NMR ($CDCl_3$, 90 MHz): δ1.30 (3H, S) 1.50 (3H, S), 3.3-3.7 (4H, m), 4.55 (1H, m), 5.1 (2H, d, J = 2), 5.65 (1H, d, J = 2), 6.1 (1H, S), 6.3-7.8 21H, M), 8.40 (1H, S).

Step b continued: $N^6$,$N^6$-Bis benzoyl-2′,3′-0-isopropylidene adenosine-5′-aldehyde.

To 2.64 g (3.73 mmol) of $N^6$,$N^6$-Bis-benzoyl-5′-deoxy-2′,3′-0-isopropylidene-5′,5′-(N,N′-diphenylethylenediamino)adenosine in 370 ml of dichloromethane at 0° C add a solution of 1.56 g (8.2 mmol) p-toluenesulfonic acid monohydrate in 180 ml of acetone. Stir the mixture for 1.5 hours and filter. Evaporate the filtrate on a rotary evaporator and partition the residue between 200 ml of dichloromethane and water. Dry the dichloromethane solution with magnesium sulfate and evaporate to a foam. Dissolve 2.10 g of the foam in 200 ml of benzene and reflux in a Dean-Stark apparatus for one hour. Evaporate the solvent to give 2.06 g of the title compound. (NMR Spectrum reveals more than 80% of the product as aldehyde.)

NMR ($CDCl_3$, 90 MHz): δ 1.40 (3H, S) 1.70 (3H, S), 4.65 (1H, S), 5.3 (1H, d, J = 7), 5.45 (1H, broad d, J = 7), 6.2 (1H, S), 7.2-7.8 (10H, m), 8.10 (1H, S), 8.45 (major) and 8.55 (1H together, two S). 9.3 (1H, S, CHO).

Step c: $N^6$,$N^6$-Bis-benzoyl-5′-deoxy-5′,5′-difluoro-2′,3′-0-isopropylideneadenosine.

Chromatograph 6.5 g of $N^6$,$N^6$-bis-benzoyl-2′,3′-0-isopropylideneadenosine-5′-aldehyde on a 40 mm x 7 cm flash silica gel column with 15% ethyl acetate/85% dichloromethane solvent. Combine and evaporate all fractions with UV -active material on Thin Layer Chromatography (TLC) to give 5.2 g of a foam. Reflux the foam in 200 ml of benzene for 2 hours and then evaporate and dry *in vacuo* to give 4.65 g of purified $N^6$,$N^6$-bis-benzoyl 2′3′-0-isopropylideneadenosine-5′-aldehyde. Dissolve 3.90 g of the 5′-aldehyde in 25 ml of dichloromethane (distilled from calcium hydride) and to this solution add 3.2 ml (3 equivalents) of diethylaminosulfur trifluoride. Stir the mixture for 6 hours. Dilute the mixture with chloroform and pour into 50 ml of stirred saturated aqueous sodium bicarbonate. Extract the product into 400 ml of chloroform and dry with $MgSO_4$. Evaporate the solvent to give 3.60 g of a foam. Percolate the product through a 40 mm x 12 cm silica gel flash column with 4% ethyl acetate/96% dichloromethane solvent. Isolate the title compound (738 mg) by TLC ($R_f$ 0.6 with 10% ethyl acetate/90% dichloromethane as solvent).

NMR ($CDCl_3$, 300 MHz): δ 1.42 (3H, S) 1.65 (3H, S) 4.42-4.53 (1H, three m), 5.27 (1H, dd, J = 2.7, 5.9), 5.39 (1H, dd, J = 1.7, 6.0), 5.96 (1H, td, J = 55, 4.5), 7.34- 7.52 (6H, m), 7.85 (4H, d J = 7.2), 8.15 (1H, S), 8.67 (1H, S).

19F-NMR ($CDCl_3$, 282 MHz, ppm from external $CFCl_3$)- 54.87 (ddd, J = 12.4, 55.2, 299.0) - 50.71 (ddd, J = 10, 55.2, 299.1)

MS (FAB - XENON) M + 1 = 536

Anal: Calc'd for $C_{27}H_{23}F_2N_5O_5$. C 60.56, H 4.33

Found: C 60.26, H 4.44

Step d: $N^6$ Benzoyl-4′,5′-didehydro-2′,3′-0-isopropylidene-5′-deoxy-5′-fluoroadenosine

To 401 mg (0.75 mmol) of crushed $N^6$,$N^6$-Bis-benzoyl-5′-deoxy-5′,5′-difluoro-2′,3′-0-isopropylideneadenosine and 335 mg (4 equivalents) of potassium t-butoxide under nitrogen add 2 ml of dimethylsulfoxide (distilled from calcium hydride). Stir the mixture under nitrogen for 21 hours. Quench with 4 ml of saturated ammonium chloride and extract with ethyl acetate to yield 274 mg of yellow oil. Percolate the oil through a 20 mm x 15 cm flash column with 30% ethyl acetate/70% dichloromethane. Combine

fractions that have two spots close together at Rf = 0.55 (TLC with ethyl acetate as solvent). Evaporate these fractions to yield 183 mg of the title compound containing two isomers in a 2:1 ratio.

NMR ($CDCl_3$, 300 MHz): δ 1.34 and 1.37 (minor) 3H together two S.), 1.49 (3H, s), 5.35-5.38 (1H, m), 5.56 and 5.90 (1H together; d, J=4 and m, resp.), 6.23 (broad s, minor) and 6.25 (1H together), 6.43 (d, J=74, major) and 6.81 (d, J=77; 1H together), 7.39-7.98 (6H, m), 8.646 (major) and 8.653 (minor; two s, 1H together), 9.05 (1H, broad, NH)

NMR $^{19}F$, 282 MHz, ppm from external $CFCl_3$): δ- 158.94 (d, J=74 major), 174.4 (d, J=77, minor) MS: (CI) M+1 = 412.

Step e: $N^6$-Benzoyl-4′,5′-didehydro-5′-deoxy-5′-fluoro adenosine

Dissolve 178 mg of $N^6$-benzoyl-4′,5′-didehydro-2′,3′-0-isopropylidene-5′-deoxy-5′-fluoroadenosine (2:1 mixture of isomers) in 2 ml of cold trifluoroacetic acid-water (4:1). Stir the mixture at room temperature for 50 minutes and then evaporate on a rotary evaporator. Chromatograph the residue on a 20 mm x 14 cm flash silica gel column with ethyl acetate as the solvent. Combine fractions to give 3 mg of the higher $R_f$ isomer (minor isomer), 58 mg of a mixture of isomers and 83 mg of the lower $R_f$ isomer (major isomer) of the title compound.

NMR ($CD_3OD$, higher $R_f$ isomer, 90 MHz): δ 5.1 (2H, m ), 6.35 (1H, d, J=6), (1H, D, J=74), 7.5-8.2 (5H, m), 8.63 (1H, s), 8.72 (1H, S).

NMR ($CD_3OD$, major lower $R_f$ isomer, 90 MHz): δ 5.00-5.10 (2H, m), 6.37 (1H, d, J=7), 6.48 (1H, a, J=75), 7.54-8.19 (5H, m), 8.53 (1H, s), 8.62 (1H, s).

Step f: (Z)-4′,5′-didehydro-5′-deoxy-5′-fluoroadenosine.

Dissolve 83 mg of $N^6$-benzoyl-4′,5′-didehydro-5′-deoxy-5′-fluoroadenosine (lower $R_f$ isomer above) in absolute ethanol, evaporate and redissolve in 6 ml of ethanol. Bubble anhydrous ammonia through the ice cooled solution in a 20 mm x 12 cm Carius tube. Seal the tube and remove the ice bath. After 14 hours at room temperature, open the tube and evaporate the solution to give 87 mg of crude product. Triturate in 1 ml of methanol and filter off the solid. Dry the product *in vacuo* to give 20 mg of the title compound (a white powder, softens at 100-110° C and melts at 225-230° C).

NMR $CD_3OD$, 300 MHz): δ 5.02-5.05 (2H, m), 6.28 (1H, d, J=F), 6.56 (1H, d, J=7.52), 8.21 (1H, s), 8.33 (1H, s).

$^{19}F$-NMR (282 MHz, ppm from external $CFCl_3$): -166.76 (d, J=75.2)

MS: (FAB-XENON) M + 1 = 268

Step f: 4′,5′-didehydro-5′-deoxy-5′-fluoroadenosine,with E-isomer as major component.

Dissolve 58 mg of $N^6$-benzoyl-4′,5′-didehydro-5′-deoxy-5′-fluoroadenosine (a mixture with the higher $R_f$ isomer being the major isomer) in 5 ml of absolute ethanol, and bubble ammonia through the ice cooled solution in a 20 mm x 12 cm Carius tube for 3 three minutes. Seal the tube and remove the ice bath. After 15 hours at room temperature, open the tube and evaporate the solution. Dissolve the residue in 2 ml of methanol and chromatograph on a 20 mm x 12 cm silica gel flash column. Eluted with ethyl acetate, followed by 10% methanol/90% ethyl acetate. Combine and evaporate fractions containing material at $R_f$ 0.23 (10% methanol/90% ethyl acetate) to yield 30 mg of product. Triturated in 12 mg of methanol and filter off the solid. Dry the product *in vacuo* to yield 16 mg of the title compound (an off-white powder). NMR indicates a 4:1 mixture of E-isomer to Z-isomer.

′H NMR (E-isomer $CD_3OD$ 300 MHz): δ5.03-5.07 (2H, m) 6.21 (1H, d, J=6.3), 7.02 (1H, d, J=78.6), 8.20 (1H, s), 8.32 (1H, s).

$^{19}F$-NMR (E-isomer, $CD_3OD$, 282 MHz, ppm from ext. $CFCl_3$): -182.30 (d, J=78.5).

MS: (CI) mH+ =268.

The following specific compounds can be made by procedures analogous to those described above in Example 3:

(E) and (Z)-5′-Bromo-4′,5′-didehydro-5′-deoxyadenosine

(E) and (Z)-5′-Chloro-4′,5′-didehydro-5′-deoxyadenosine

(E) and (Z)-5′-Bromo-4′,5′-didehydro-2′,5′-dideoxyadenosine

(E) and (Z)-5′-Chloro-4′,5′-didehydro-2′,5′-dideoxyadenosine
(E) and (Z)-5′-Fluoro-4′,5′-didehydro-2′,5′-dideoxyadenosine.

These adenosine derivatives can then be converted to the appropriate inosine/guanosine derivatives of formula (1) by the utilization of the procedure as described in Scheme A to yield the following specific compounds:

(E) and (Z)-5′-Bromo-4′,5′-didehydro-5′-deoxyinosine
(E) and (Z)-5′-Chloro-4′,5′-didehydro-5′-deoxyinosine
(E) and (Z)-5′-Fluoro-4′,5′-didehydro-5′-deoxyinosine
(E) and (Z)-5′-Bromo-4′,5′-didehydro-2′,5′-dideoxyinosine
(E) and (Z)-5′-Chloro-4′,5′-didehydro-2′,5′-dideoxyinosine
(E) and (Z)-5′-Fluoro-4′,5′-didehydro-2′,5′-dideoxyinosine

The 4′-vinylhalo-inosine/guanosine derivatives of the formula (1), wherein one of $X_1$ and $X_2$ is hydrogen and the other is halogen, can alternately be prepared by a procedure as described in Scheme C, wherein all terms are as previously defined and the term "4-MeO-φ " refers to a 4-methoxyphenyl group.

SCHEME C

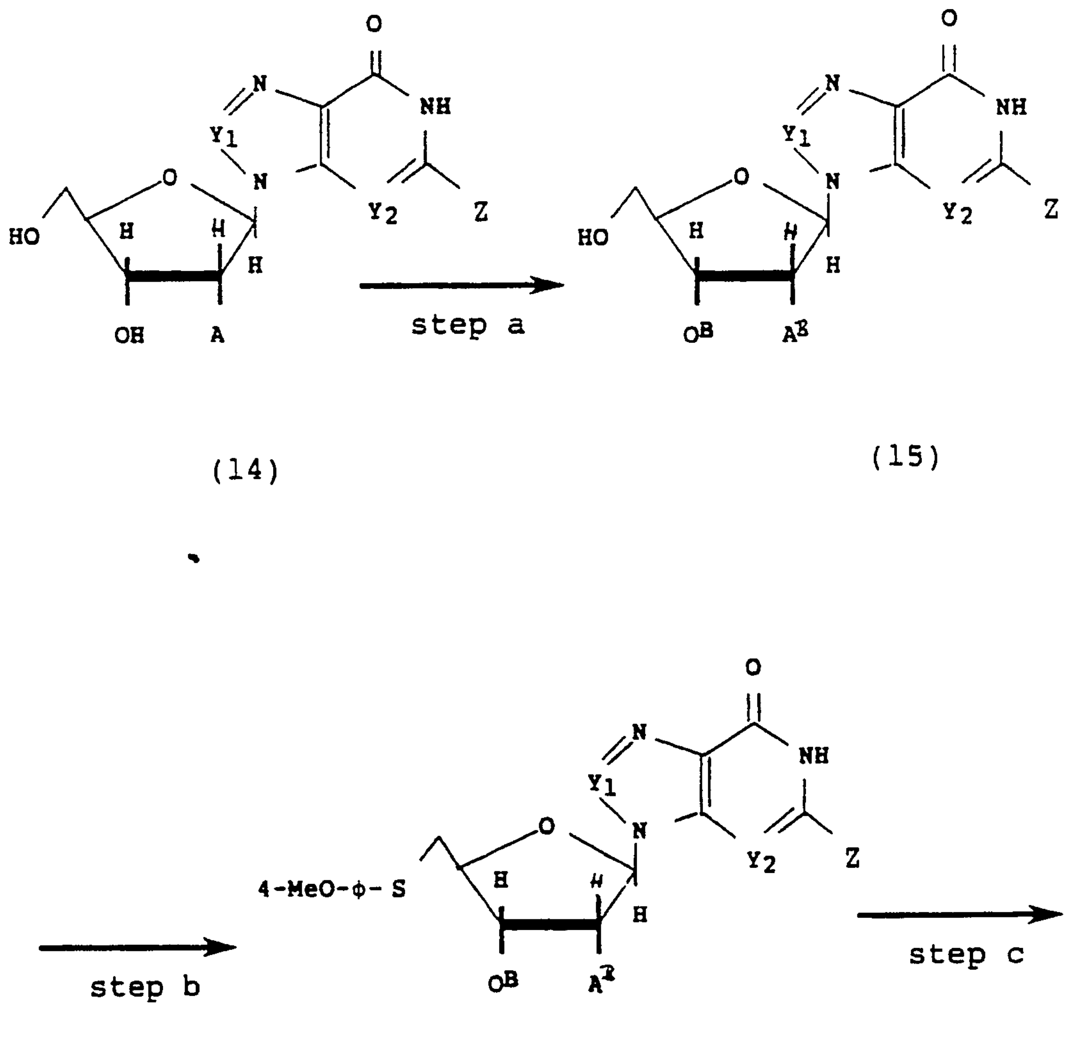


(14)

(15)

(16)

Scheme C (cont'd)

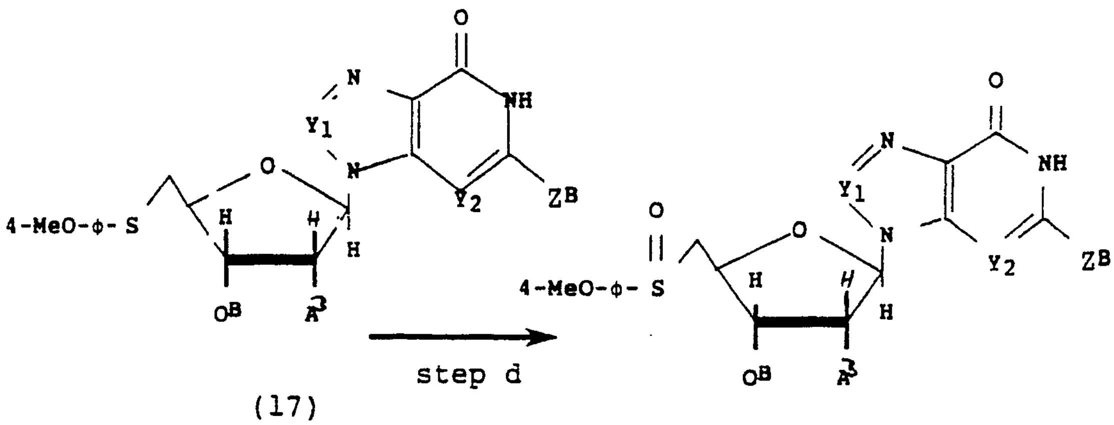


(17)

(18)

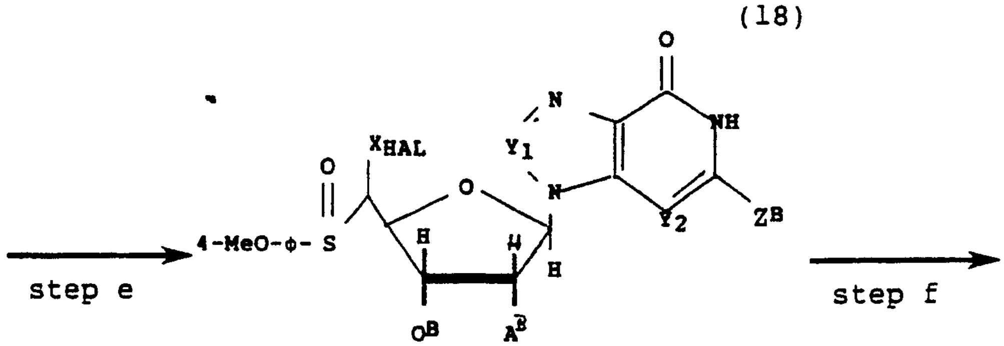


(19)

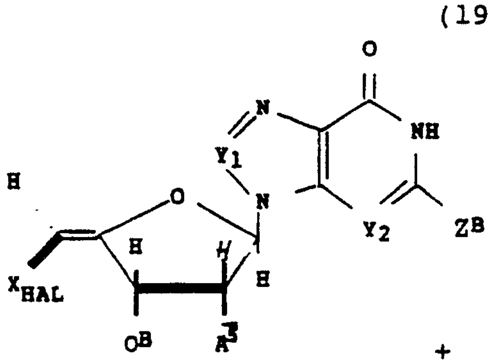

+

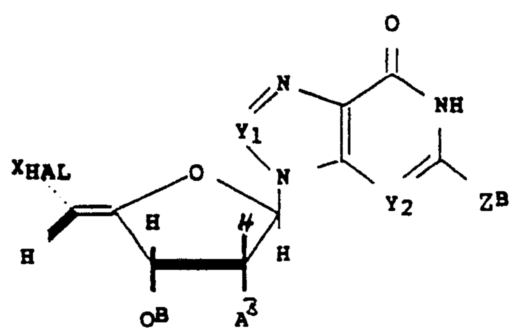

(20)

(21)

Scheme C (cont'd)

(20) (21)

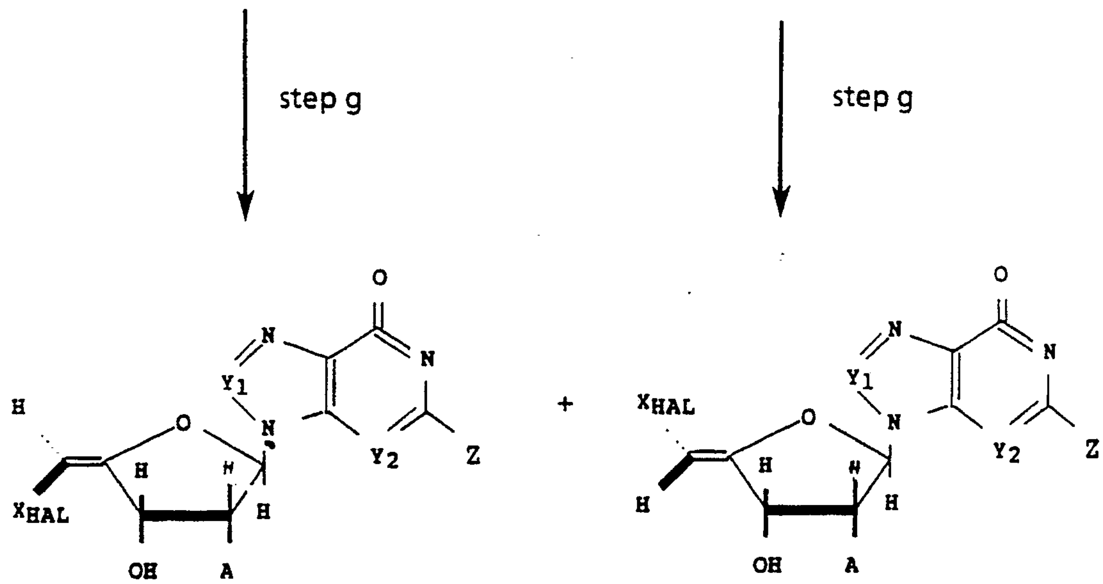


(12) (13)

In step a, reactive hydroxy groups of the appropriate 30 inosine/guanosine derivative (14), other than the 5'-hydroxy, are blocked utilizing standard blocking agents well known in the art as described for Scheme B. Where A is hydroxy, it is preferred to block the 2'- and 3'-hydroxy groups with a 2',3'-O-isopropylidene blocking group. Where A is not hydroxy, it is preferred to block the 3'-hydroxy with a benzoyl group. Where a 2',3'-O-isopropylidene blocking group is not utilized, it is preferred to block the 3'-hydroxy and any 2'-hydroxy (wherein A is hydroxy) after the reaction described in step b.

In step b, the 5'-hydroxy of the appropriately blocked 5'-hydroxy derivative (15) is subjected to a substitution reaction in which an alkyl-thio group replaces the 5'-hydroxy group to form the corresponding sulfide (16). The preferred sulfide is the 4-methoxyphenylsulfide which can be formed by reacting the appropriately blocked 5'-hydroxy derivative (15) with 4-methoxyphenyl disulfide in the presence of tributylphosphine.

In step c, any reactive amino groups, such as the 2-amino group of guanine or deoxyguanine, can be blocked as described for Scheme B. It is preferred to block the 2-amino group of guanine or deoxyguanine with an acetyl group. In addition, Where a 2',3'-O-isopropylidene blocking group is not utilized in step a, the 3'-hydroxy and any 2'-hydroxy (wherein A is hydroxy) can be blocked as described above.

In step d, the appropriately blocked sulfide (17) is oxidized to the corresponding sulfinyl derivative (18) utilizing standard oxidizing agents well known and appreciated in the art such as 3-chloroperbenzoic acid.

In step e, the 5'-carbon of the sulfinyl derivative (18) is halogenated using a halogenating agent, such as the fluorinating agent diethylaminosulfur trifluoride (DAST), or the chlorinating agent sulfuryl chloride in the presence of a base such as pyridine, to yield the corresponding 5'-halo-sulfinyl derivative (19). The preferred fluorinating agent is DAST and the preferred chlorinating agent is sulfuryl chloride. Where DAST is utilized as the fluorinating agent, the fluorinated product must be re-oxidized after treatment with DAST with an equimolar amount of an oxidizing agent, such as 3-chloroperbenzoic acid, in order to provide the 5'-halo-sulfinyl derivative (19).

In step f, the sulfinyl group is then eliminated to yield the appropriately blocked 4'-vinylhalo derivatives (20 and 21) by heating the 5'-halo-sulfinyl derivative (19) in the presence of a base such as diisopropylethyl

amine.

In step g, the blocking groups of the appropriately blocked 4′-vinylhalo derivatives (20 and 21) are removed according to conventional procedures and techniques well known and appreciated in the art such as those described for Scheme B. The (Z) and (E) isomers of the 4′-vinylhalo-inosine/guanosine derivative or the 4′-vinylhalo-deoxyinosine/guanosine derivative, i.e., (12) and (13), are thus formed. These isomers can be separated by conventional resolution techniques well known and appreciated in the art.

Starting materials for use in the general synthetic procedure outlined in Scheme C are readily available to one of ordinary skill in the art. For example, certain starting materials for various compounds of formula (1) wherein A is H or OH, $Y_1$ is CH, $Y_2$ is N and Z is H are commercially available. Additional starting materials can be prepared by the use of conventional methods and analogous techniques which are well known and appreciated in the art.

The following example presents a typical synthesis as described by Scheme C. This example is understood to be illustrative only and is not intended to limit the scope of the present invention in any way.

## EXAMPLE 4

### (Z)- and (E)-4′,5′-Didehydro-5′-deoxy-5′-fluoroguanosine

#### Step a: 2′,3′-O-isopropylidene-guanosine

The title compound is commercially available.

#### Step b: 5′-Deoxy-2′,3′-O-isopropylidene-5′-[(4-methoxyphenyl)thio]guanosine

To a mixture of 2′,3′-O-isopropylideneguanosine (16.1 g, 0.05 moles) and 4-methoxyphenyl disulfide (26.0 g, 0.094 moles) in dry pyridine (125 mL), add tributylphosphine (23.3 mL, 0.094 moles) via syringe. Stir under nitrogen overnight. Add water and remove solvents *in vacuo* in a rotary evaporator. Stir the residue in a mixture of water and cyclohexane. Decant the cyclohexane layer. Repeat the cyclohexane extraction two more times. Extract the water layer with ethyl acetate. Dry the ethyl acetate solution with $MgSO_4$ and concentrate to 100 mL or less. Add chloroform to the ethyl acetate solution and cool. Collect crystals of the title compound.

#### Step c: $N^2$-acetyl-5′-Deoxy-2′,3′-O-isopropylidene-5′-[(4-methoxyphenyl)thio]guanosine

To 5′-Deoxy-2′,3′-O-isopropylidene-5′-[(4-methoxyphenyl)thio]guanosine (10 g, 0.022 moles) in 50 mL of pyridine, add 4.1 mL (2 equivalents) of acetic anhydride and stir overnight. Pour the mixture into water and extract with ethyl acetate. Dry the ethyl acetate extract with $MgSO_4$ and remove the solvents *in vacuo* to give the title compound.

#### Step d: $N^2$-acetyl-5′-Deoxy-2′,3′-O-isopropylidene-5′-[(4-methoxyphenyl)sulfoxyl]guanosine

To a solution of $N^2$-acetyl-5′-deoxy-2′,3′-O-isopropylidene-5′-[(4-methoxyphenyl)thio]guanosine (5 g, 0.01 moles) in 40 mL dichloromethane cooled in an ice bath, add dropwise a solution of 2.0 g (0.01 moles) of 85% 3-chloroperbenzoic acid in 40 mL dichloromethane. Stir the reaction mixture for 2 hours and partition the mixture between 400 mL chloroform and a saturated aqueous sodium bicarbonate solution. Dry the organic layer over $MgSO_4$ and evaporate the solution to dryness. Chromatograph the residue on 200 g silica gel eluting with ethyl acetate/methanol. Concentrate the eluant to yield the title compound.

#### Step e: $N^2$-acetyl-5′-Deoxy-5′-dehydro-2′,3′-O-isopropylidene-5′-fluoro-5′-[4-methoxyphenyl sulfoxyl]-guanosine

To 3.0 g of $N^2$-acetyl-5′-deoxy-2′,3′-O-isopropylidene-5′-[4-methoxyphenylsulfoxyl]guanosine (5.96 mmol) in 15 mL of 1,2-dichloroethane, add 3.15 mL (23.8 mmol) of diethylaminosulfur trifluoride. Stir the reaction mixture at 45° C for 2 hours, cool and pour into saturated aqueous sodium bicarbonate. Extract the mixture with 200 mL chloroform. Dry the extract over $MgSO_4$ and evaporate the solvents *in vacuo*. Redissolve the residue in dichloromethane and evaporate the solvents *in vacuo*. Dissolve the residue in 25 mL of dichloromethane and cool in an ice bath. To this solution, add dropwise a solution of 0.97 g of 85% 3-chloroperbenzoic acid and stir for 2 hours. Partition the reaction mixture between 200 mL chloroform and saturated aqueous sodium bicarbonate. Dry the organic layer over $MgSO_4$ and evaporate the solvents to dryness. Chromatograph the residue on 100 g silica gel eluting sequentially with cyclohexane/ethyl acetate, ethyl acetate and ethyl acetate/methanol to provide the title compound.

Step f: (Z)- and (E)-$N^2$-acetyl-5′-Deoxy-4′,5′-didehydro-2′,3′-O-isopropylidene-5′-fluoroguanosine

Reflux a solution of 1.8 g of $N^2$-acetyl-5′-deoxy-5′-dehydro-2′,3′-O-isopropylidene-5′-fluoro-5′-[4-methoxyphenylsulfoxyl]guanosine in 35 mL diglyme and 2.5 g diisopropylethylamine for 24 hours. Remove solvents by distillation on a Kugelrohr apparatus at 1 mm Hg. Chromatograph the residue on 50 g silica gel eluting sequentially with cyclohexane/ethyl acetate, ethyl acetate and ethyl acetate/methanol to provide a mixture of the (Z) and (E) isomers of the title compound.

Step g: (Z)- and (E)-5′-Deoxy-4′,5′-didehydro-5′-fluoroguanosine

Place 0.5 g of (Z)- and (E)-$N^2$-acetyl-5′-deoxy-4′,5′-didehydro-2′,3′-O-isopropylidene-5′-fluoroguanosine (0.136 mmol) in 15 mL ethanol, cool to 0° C and saturate with ammonia in a pressure tube. Cap the tube and keep it at room temperature overnight. Evaporate the solvent and stir the residue in a few mL of ethyl acetate. Filter the mixture and dissolve the filter cake in 5 mL of trifluoroacetic acid/water (4/1, v/v). Stir the mixture at room temperature for 1 hour and evaporate *in vacuo*. Stir the residue in a few mL of ethyl acetate and filter to afford a 2:1 mixture of the (Z) and (E) isomers, respectively, of the title compound. Separate the isomers on a Bio-Rad® AG 1-1X resin (OH-form) under conditions described for guanosine by Dekker [*J.Am. Chem. Soc.* 87 , 4027-29 (1965)].

The following specific compounds can be made by procedures analogous to those described in Example 8:

(Z) and (E)-4′,5′-Didehydro-5′-deoxy-5′-chloro-guanosine
(Z) and (E)-4′,5′-Didehydro-5′-deoxy-5′-chloro-inosine
(Z) and (E)-4′,5′-Didehydro-2′,5′-dideoxy-5′-chloro-guanosine
(Z) and (E)-4′,5′-Didehydro-2′,5′-dideoxy-5′-chloro-inosine
(Z) and (E)-4′,5′-Didehydro-5′-deoxy-5′-fluoro-inosine
(Z) and (E)-4′,5′-Didehydro-2′,5′-dideoxy-5′-fluoro-guanosine
(Z) and (E)-4′,5′-Didehydro-2′,5′-dideoxy-5′-fluoro-inosine.

The 4′-vinylhalo-inosine/guanosine derivatives of the formula (1) wherein both of $X_1$ and $X_2$ are halogen, can be prepared according to the procedure described in Scheme D. This procedure can be utilized to provide compounds of formula (1) wherein $X_1$ and $X_2$ are the same halogen, as well as to provide compounds of formula (1) wherein $X_1$ and $X_2$ are different halogens. This procedure is particularly useful to provide compounds of formula (1) wherein both $X_1$ and $X_2$ are chlorine atoms and wherein one of $X_1$ and $X_2$ is a fluorine atom and the other is a chlorine atom.

Scheme D

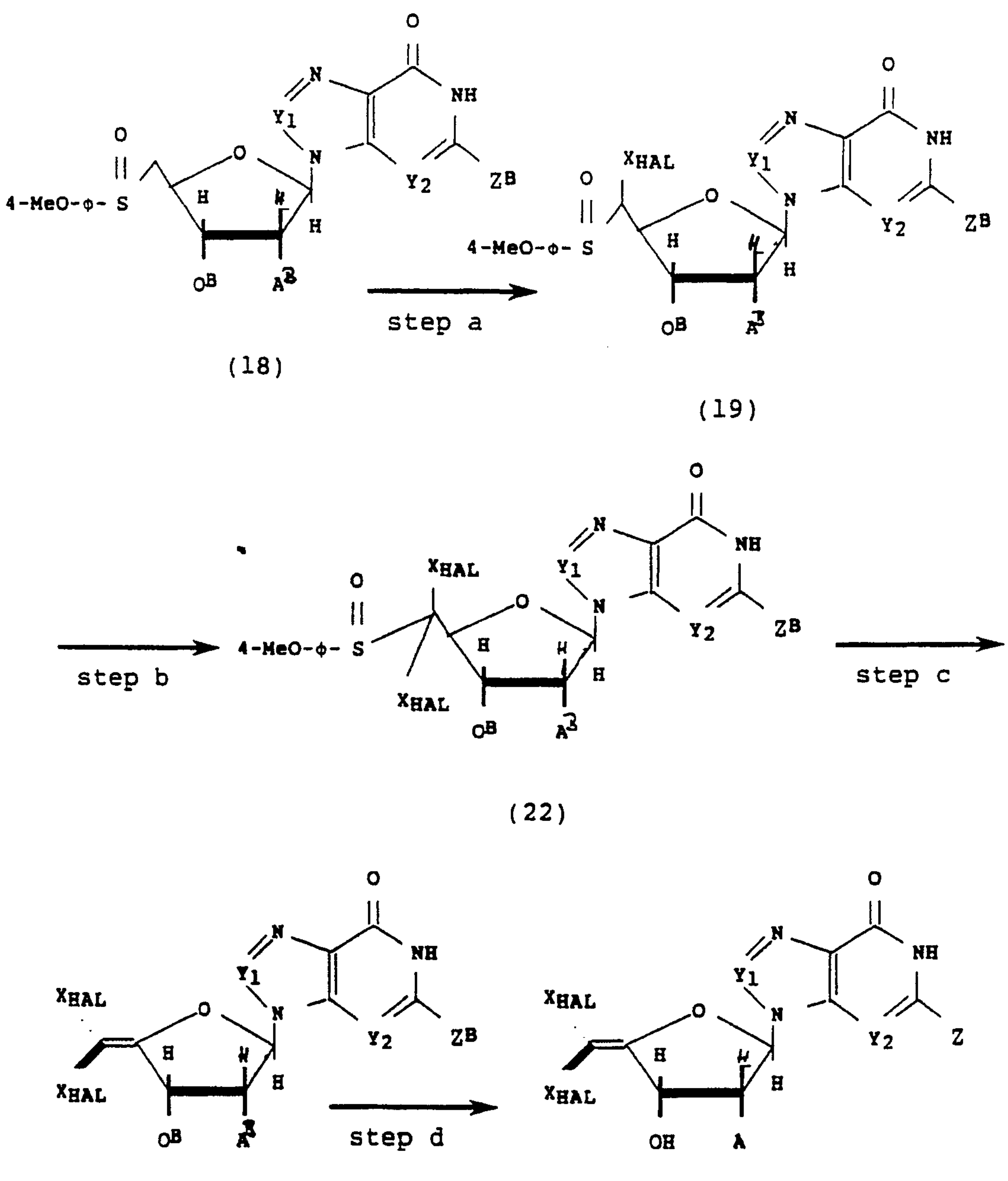


(18) (19) (22) (23) (24)

In step a, the 5′-carbon of the sulfinyl derivative (18), which is prepared as described in Scheme C, is halogenated using a halogenating agent to yield the corresponding 5′-halo-sulfinyl derivative (19). Where fluorination is desired, it is preferred to treat the sulfinyl derivative (18) with the fluorinating agent DAST followed by re-oxidation with 3-chloroperbenzoic acid as described for Scheme C. Where chlorination is desired, it is preferred to treat the sulfinyl derivative (18) with the chlorinating agent sulfuryl chloride in the presence of a base such as pyridine.

In step b, halogenation of the 5′-carbon of the 5′-halo-sulfinyl derivative (19) is continued so as to provide a 5′,5′-dihalo-sulfinyl derivative (22). Where compounds of formula (1) are desired wherein $X_1$ and $X_2$ are both chlorine atoms, the sulfinyl derivative (18), depicted in step a, is treated with 2 equivalents of sulfuryl chloride to yield the corresponding 5′,5′-dichloro-sulfinyl derivative. Where compounds of formula

(1) are desired wherein $X_1$ and $X_2$ are different halogens, the sulfinyl derivative (18) is treated with 1 equivalent of the appropriate halogenating agent. The 5′-halo-sulfinyl derivative (19) is isolated and then treated with 1 equivalent of the appropriate different halogenating agent.

For example, where compounds of formula (1) are desired wherein one of $X_1$ and $X_2$ is a fluorine atom and the other is a chlorine atom, the sulfinyl derivative (18) is treated with 1 equivalent of a fluorinating agent such as DAST, and re-oxidised as described above, to yield the corresponding 5′-fluoro-sulfinyl derivative. The 5′-fluoro-sulfinyl derivative is then chlorinated with 1 equivalent of a chlorinating agent such as sulfuryl chloride to yield the corresponding 5′-fluoro-5′-chloro-sulfinyl derivative. For example, the 5′-fluoro-sulfinyl derivative (19) can be reacted with sulfuryl chloride in dichloromethane in the presence of pyridine.

In step c, the sulfinyl group of the 5′,5′-dihalo-sulfinyl derivative (22) is eliminated as described in Scheme C, step f, to yield the appropriately blocked 4′-vinyl-dihalo derivative (23).

In step d, the blocking groups of the appropriately blocked 4′-vinyl-dihalo derivative (23) are removed as described in Scheme C, step g, to yield the 4′-vinyl-dihalo derivative 24.

It is of course understood that where $X_1$ and $X_2$ are each different halogens, the 4′-vinyl-dihalo derivative (24) will exist as a mixture of the (Z) and (E) isomers. These isomers can be readily separated by conventional techniques and procedures as are well known and appreciated in the art.

The following example presents a typical synthesis as described by Scheme C. This example is understood to be illustrative only and is not intended to limit the scope of the present invention in any way.

## EXAMPLE 5

### (Z)- and (E)-4′,5′-Didehydro-5′-deoxy-5′-fluoro-5′-chloro-guanosine

Step a: $N^2$-acetyl-5′-Deoxy-2′,3′-O-isopropylidene-5′-fluoro-5′-[4-methoxyphenylsulfoxyl]guanosine

To 3.0 g of $N^2$-acetyl-5′-deoxy-2′,3′-O-isopropylidene-5′-[4-methoxyphenylsulfoxyl]guanosine (5.96 mmol) in 15 mL of 1,2-dichloroethane, add 3.15 mL (23.8 mmol) of diethylaminosulfur trifluoride. Stir the reaction mixture at 45° C for 2 hours, cool and pour into saturated aqueous sodium bicarbonate. Extract the mixture with 200 mL chloroform. Dry the extract over $MgSO_4$ and evaporate the solvents *in vacuo*. Redissolve the residue in dichloromethane and evaporate the solvents *in vacuo*. Dissolve the residue in 25 mL of dichloromethane and cool in an ice bath. To this solution, add dropwise a solution of 0.97 g of 85% 3-chloroperbenzoic acid and stir for 2 hours. Partition the reaction mixture between 200 mL chloroform and saturated aqueous sodium bicarbonate. Dry the organic layer over $MgSO_4$ and evaporate the solvents to dryness. Chromatograph the residue on 100 g silica gel eluting sequentially with cyclohexane/ethyl acetate, ethyl acetate and ethyl acetate/methanol to provide the title compound.

Step b: $N^2$-acetyl-5′-Deoxy-2′,3′-O-isopropylidene-5′-fluoro-5′-chloro-5′-[4-methoxyphenylsulfoxyl]guanosine

To $N^2$-acetyl-5′-deoxy-2′,3′-O-isopropylidene-5′-fluoro-5′-[4-methoxyphenylsulfoxyl]guanosine (0.89g, 1.7 mmol) in 6.5 mL of dry dichloromethane add 0.32 mL (4.0 mmol) of pyridine and cool the mixture in an ice bath under nitrogen. To this mixture, add 0.18 mL (1.9 mmol) of $SO_2Cl_2$ (sulfuryl chloride) and stir the mixture for 20 minutes. Remove the solvents *in vacuo* to afford a foam. Percolate the product through a silica gel column eluting with dichloromethane followed by ethyl acetate/dichloromethane (1:4, v/v). Repeat the percolation procedure to yield the title compound as a foam. Triturate the product with dichloromethane/cyclohexane to yield a solid.

Step c: (Z)- and (E)-$N^2$-acetyl-5′-Deoxy-4′,5′-didehydro-2′,3′-O-isopropylidene-5′-fluoro-5′-chloro-guanosine

Reflux a solution of 1.0 g of $N^2$-acetyl-5′-deoxy-2′,3′-O-isopropylidene-5′-fluoro-5′-chloro-5′-[4-methoxyphenylsulfoxyl]guanosine in 35 mL diglyme and 2.5 g diisopropylethylamine for 24 hours. Remove solvents by distillation on a Kugelrohr apparatus at 1 mm Hg. Chromatograph the residue on 50 g silica gel

eluting sequentially with cyclohexane/ethyl acetate, ethyl acetate and ethyl acetate/methanol to provide a mixture of the (Z) and (E) isomers of the title compound.

Step d: (Z)- and (E)-5′-Deoxy-4′,5′-didehydro-5′-fluoro-5′-chloro-guanosine

Place 0.4 g of (Z)- and (E)-$N^2$-acetyl-5′-deoxy-4′,5′-didehydro-2′,3′-O-isopropylidene-5′-fluoro-5′-chloro-guanosine (1.0 mmol) in 15 mL ethanol, cool to 0° C and saturate with ammonia in a pressure tube. Cap the tube and keep it at room temperature overnight. Evaporate the solvent and stir the residue in a few mL of ethyl acetate. Filter the mixture and dissolve the filter cake in 5 mL of trifluoroacetic acid/water (4/1, v/v). Stir the mixture at room temperature for 1 hour and evaporate *in vacuo*. Stir the residue in a few mL of ethyl acetate and filter to afford a mixture of the (Z) and (E) isomers of the title compound. Separate the isomers on a Bio-Rad® AG 1-1X resin (OH-form) under conditions described for guanosine by Dekker [*J. Am. Chem. Soc.* 87, 4027-29 (1965)].

The following specific compounds can be made by procedures analogous to those described in Example 8:

(Z)- and (E)-5′-Deoxy-4′,5′-didehydro-5′-fluoro-5′-chloro-inosine
(Z)- and (E)-2′,5′-Dideoxy-4′,5′-didehydro-5′-fluoro-5′-chloro-inosine
(Z)- and (E)-2′,5′-Dideoxy-4′,5′-didehydro-5′-fluoro-5′-chloro-guanosine
(Z)- and (E)-5′-Deoxy-4′,5′-didehydro-5′,5′-dichloro-inosine
(Z)- and (E)-2′,5′-Dideoxy-4′,5′-didehydro-5′,5′-dichloro-inosine
(Z)- and (E)-5′-Deoxy-4′,5′-didehydro-5′,5′-dichloro-guanosine
(Z)- and (E)-2′,5′-Dideoxy-4′,5′-didehydro-5′,5′-dichloro-guanosine.

The present invention provides a method of treating a patient suffering from a neoplastic disease comprising administering to said patient a therapeutically effective antineoplastic amount of a compound of formula (1). By administering a therapeutically effective antineoplastic amount of a compound of formula (1) to a patient suffering from a neoplastic disease, an antineoplastic effect is provided. It is believed that this antineoplastic effect is generated through an inhibition of purine nucleoside phosphorylase, however this invention is not limited by the proposed mechanism by which it occurs.

The term "neoplastic disease" as used herein refers to an abnormal state or condition characterized by rapidly proliferating cell growth or neoplasm. Based upon standard laboratory experimental techniques and procedures well known and appreciated by those skilled in the art, as well as upon comparisons with compounds of known usefulness, the compounds of formula (I) are useful in the treatment of patients suffering from those neoplastic diseases which are dependent upon purine nucleoside phosphorylase for their growth. Such neoplastic diseases include: leukemias, including but not limited to acute lymphoblastic, chronic lymphocytic, acute myloblastic and chronic mylocytic; carcinomas, including but not limited to those of the cervix, esophagus, stomach, small intestines, colon and lungs; sarcomas, including but not limited to oesteroma, osteosarcoma, lipoma, liposarcoma, hemangioma and hemangiosarcoma; melanomas, including amelanotic and melanotic; and mixed types of neoplasias such as, for example, carcinosarcoma, lymphoid tissue type, folicullar reticulum, cell sarcoma and Hodgkins Disease. Of course, one skilled in the art will recognize that not every compound of formula (I) will be effective against each of the neoplastic disease states, and that selection of the most appropriate compound is within the ability of one of ordinary skill in the art and will depend on a variety of factors including assessment of results obtained in standard animal tumor models.

The term "antineoplastic effect" refers to an effect of controlling the growth or proliferation of the neoplasm or in prolonging the survivability of the patient beyond that expected in the absence of such treatment. The growth or proliferation of a neoplasm is controlled by slowing, interrupting, arresting or stopping its growth, proliferation or its metastases. The term "controlling the growth or proliferation of the neoplasm" therefore does not necessarily indicate a total elimination of the neoplasm. It is believed that prolonging the survivability of a patient, beyond being a significant advantageous effect in and of itself, also indicates that the growth of the neoplasm has been controlled.

A neoplastic disease is dependent upon purine nucleoside phosphorylase when purine nucleoside phosphorylase activity is required within the rapidly proliferating cells or neoplasm for their growth, proliferation or metastases.

As used herein, the term "patient" refers to a warm-blooded animal, such as a mammal, which is suffering from a neoplastic disease which is dependent on purine nucleoside phosphorylase. It is understood that humans are included within the scope of the meaning of the term.

In effecting treatment of a patient afflicted with a neoplastic disease described above, a compound of

formula (1) can be administered in any form or mode which makes the compound bioavailable in therapeutically effective antineoplastic amounts, including oral and parenteral routes. For example, compounds of formula (1) can be administered orally, topically, subcutaneously, intramuscularly, intravenously, transdermally, intranasally, rectally, and the like. Oral administration is generally preferred. One skilled in the art can readily select the proper form and mode of administration depending upon the particular characteristics of the compound selected the disease state to be treated, the stage of the disease, and other relevant circumstances.

As used herein, the term "therapeutically effective antineoplastic amount" refers to an amount of the compound of formula (1) which is effective in providing an antineoplastic effect. A therapeutically effective antineoplastic amount can be readily determined by the attending diagnostician, as one skilled in the art, by the use of known techniques and by observing results obtained under analogous circumstances. In determining the therapeutically effective antineoplastic amount, a number of factors are considered by the attending diagnostician, including, but not limited to: the species of mammal; its size, age, and general health; the specific disease involved; the degree of or involvement or the severity of the disease; the response of the individual patient; the partioular compound administered; the mode of administration; the bioavailability characteristics of the preparation administered; the dose regimen selected; the use of concomitant medication; and other relevant circumstances.

A therapeutically effective antineoplastic amount of a compound of formula (1) is expected to vary from about 0.1 milligram per kilogram of body weight per day (mg/kg/day) to about 100 mg/kg/day. Preferred amounts are expected to vary from about 1 to about 20 mg/kg/day. These ranges are particularly reflective of effective amounts upon oral administration but also are reflective of the operable ranges for parenteral administration.

The compounds can be administered alone or in the form of a pharmaceutical composition in combination with pharmaceutically acceptable carriers or excipients, the proportion and nature of which are determined by the solubility and chemical properties of the compound selected, the chosen route of administration, and standard pharmaceutical practice. The compounds of formula (1), while effective themselves, may be formulated and administered in the form of their pharmaceutically acceptable acid addition salts for purposes of stability, convenience of crystallization, increased solubility and the like.

The compounds of formula (1) can be provided in compositions comprising an assayable amount of a compound of formula (1) in admixture with one or more inert carriers. These compositions are useful, for example, as assay standards, as convenient means of making bulk shipments, or as pharmaceutical compositions. An assayable amount of a compound of formula (1) is an amount which is readily measurable by standard assay procedures and techniques as are well known and appreciated by those skilled in the art. Assayable amounts of a compound of formula (1) will generally vary from about 0.001% to about 75% of the composition by weight. Inert carriers can be any material which does not degrade or otherwise covalently react with a compound of formula (1). Examples of suitable inert carriers are water; aqueous buffers, such as those which are generally useful in High Performance Liquid Chromatography (HPLC) analysis; organic solvents, such as acetonitrile, ethyl acetate, hexane and the like; and pharmaceutically acceptable carriers or excipients. These compositions are prepared by mixing the compound of formula (1) with the inert carriers utilizing techniques and methods which are well known and appreciated in the art.

More particularly, compounds of formula (1) can be provided in pharmaceutical compositions comprising a therapeutically effective antineoplastic amount of a compound of formula (1) in admixture or otherwise in association with one or more pharmaceutically acceptable carriers or excipients.

The pharmaceutical compositions are prepared in a manner well known in the pharmaceutical art. The carrier or excipient may be a solid, semi-solid, or liquid material which can serve as a vehicle or medium for the active ingredient. Suitable carriers or excipients are well known in the art. The pharmaceutical composition may be adapted for oral or parenteral use and may be administered to the patient in the form of tablets, capsules, suppositories, solution, suspensions, or the like.

The compounds of formula (1) may be administered orally, for example, with an inert diluent or with an edible carrier. They may be enclosed in gelatin capsules or compressed into tablets. For the purpose of oral therapeutic administration, the compounds may be incorporated with excipients and used in the form of tablets, troches, capsules, elixirs, suspensions, syrups, wafers, chewing gums and the like. These preparations should contain at least 4% of the compound of the invention, the active ingredient, but may be varied depending upon the particular form and may conveniently be between 4% to about 70% of the weight of the unit. The amount of the compound present in compositions is such that a suitable dosage will be obtained. Preferred compositions and preparations according to the present invention are prepared so that an oral dosage unit form contains between 5.0-300 milligrams of a compound of the invention.

The tablets, pills, capsules, troches and the like may also contain one or more of the following

adjuvants: binders such as microcrystalline cellulose, gum tragacanth or gelatin; excipients such as starch or lactose, disintegrating agents such as alginic acid, Primogel, corn starch and the like; lubricants such as magnesium stearate or Sterotex; glidants such as colloidal silicon dioxide; and sweetening agents such as sucrose or saccharin may be added or a flavoring agent such as peppermint, methyl salicylate or orange flavoring. When the dosage unit form is a capsule, it may contain, in addition to materials of the above type, a liquid carrier such as polyethylene glycol or a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the present compounds, sucrose as a sweetening agent and certain preservatives, dyes and colorings and flavors. Materials used in preparing these various compositions should be pharmaceutically pure and nontoxic in the amounts used.

For the purpose of parenteral therapeutic administration, the compounds of formula (1) may be incorporated into a solution or suspension. These preparations should contain at least 0.1% of a compound of formula (1), but may be varied to be between 0.1 and about 50% of the weight thereof. The amount of the compound present in such compositions is such that a suitable dosage will be obtained. Preferred compositions and preparations according to the present invention are prepared so that a parenteral dosage unit contains between 5.0 to 100 milligrams of the compound of the invention.

The solutions or suspensions may also include the one or more of the following adjuvants: sterile diluents such as water for injection, saline solution, fixed oils, polyethylene glycols, glycerine, propylene glycol or other synthetic solvents; antibacterial agents such as benzyl alcohol or methyl paraben; antioxidants such as ascorbio acid or sodium bisulfite; chelating agents such as ethylene diaminetetraacetic acid; buffers such as acetates, citrates or phosphates and agents for the adjustment of tonicity such as sodium chloride or dextrose. The parenteral preparation can be enclosed in ampules, disposable syringes or multiple dose vials made of glass or plastic.

In addition, the present invention also provides a method of inhibiting a purine nucleoside phosphorylase in a neoplasm of a patient suffering from a neoplastic disease which is dependent upon purine nucleoside phosphorylase for its growth, comprising administering to said patient an effective purine nucleoside phosphorylase inhibitory amount of a compound of formula (1). By administering an effective purine nucleoside phosphorylase inhibitory amount of a compound of formula (1), the purine nucleoside phosphorylase in the neoplasm is inhibited, thereby providing an antineoplastic effect. Compounds of formula (1) are administered as described above. The term "effective purine nucleoside phosphorylase inhibitory amount" refers to that amount of a compound of formula (1) which is effective in substantially inhibiting the purine nucleoside phosphorylase in the neoplasm of the patient so as to provide an antineoplastic effect. An effective purine nucleoside phosphorylase inhibitory amount is the same as a therapeutically effective antineoplastic amount as described above.

As with any group of structurally related compounds which possesses a particular generic utility, certain groups and configurations are preferred for compounds of formula (1) in their end-use application..

With respect to the substituents $X_1$ and $X_2$, compounds of formula (1) wherein $X_1$ is fluoro and $X_2$ is hydrogen, and those wherein $X_1$ is hydrogen and $X_2$ is fluoro, are generally preferred. With respect to the substituent $A_1$, compounds of formula (1) wherein $A_1$ is hydrogen are generally preferred. With respect to the substituent $A_2$, compounds of formula (1) wherein $A_2$ is hydroxy are generally preferred. Furthermore, compounds of formula (1) wherein $Y_1$ is a CH group and $Y_2$ is nitrogen are generally preferred.

## Claims

1. A compound of formula 1

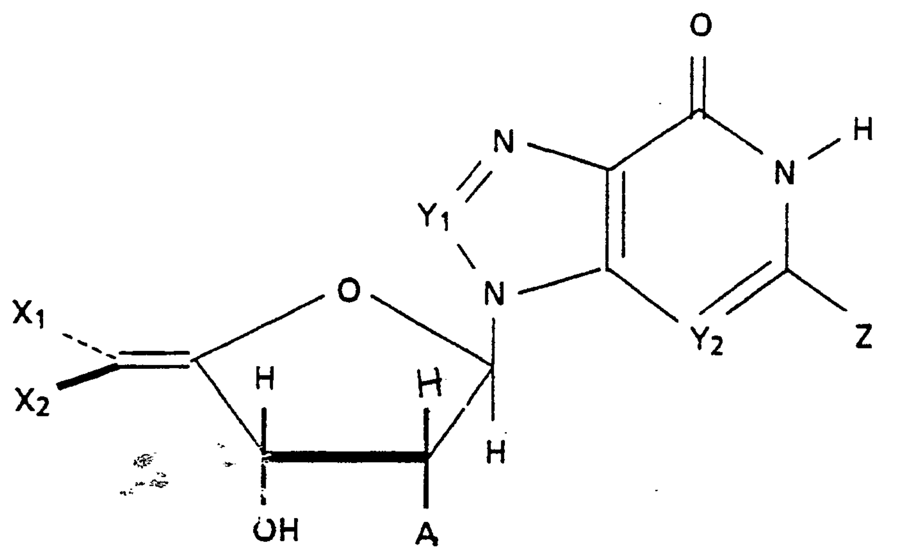

(1)

wherein

$X_1$ and $X_2$ are each independently hydrogen or halogen with the proviso that at least one of $X_1$ and $X_2$ is a halogen atom,

A is hydrogen or hydroxy,

$Y_1$ is nitrogen, a CH group, a CCl, a CBr group or a $CNH_2$ group,

$Y_2$ is nitrogen or a CH group, and

Z is hydrogen, halogen, or $NH_2$.

2. A process for preparing the compounds of formula 1 according to claim 1 wherein

Z is other than $NH_2$

comprising reacting an acidic oxidative agent with a compound of the formula 2

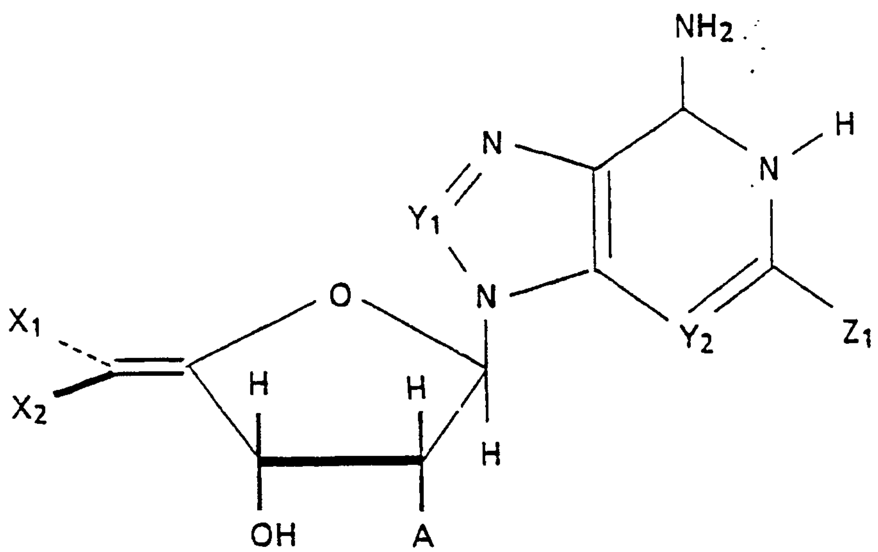

(2)

wherein the substituents $X_1$, $X_2$, A, $Y_1$ and $Y_2$ have the same meaning as in claim 1 and $Z_1$ is hydrogen or halogen.

3. A process for preparing the compounds of formula 1 according to claim 1 comprising reacting a compound of the formula

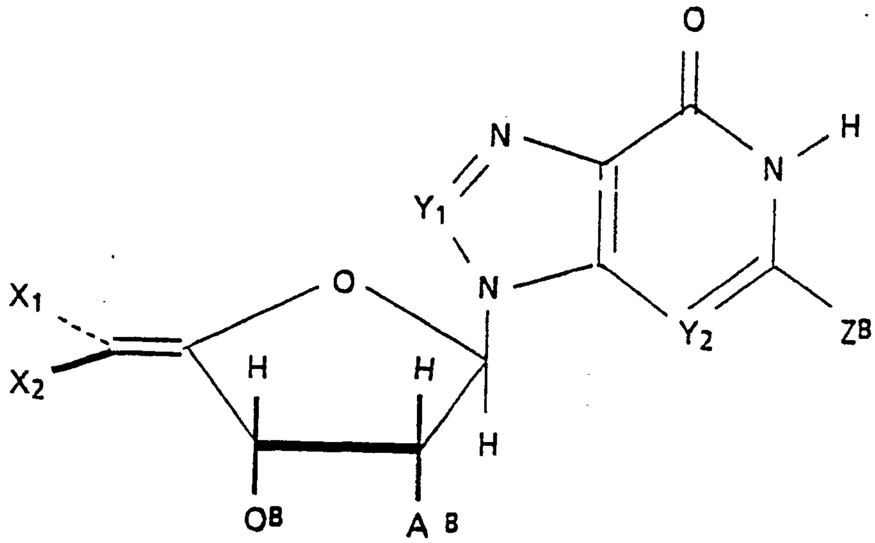

wherein $X_1$, $X_2$, $Y_1$ and $Y_2$ have the same meaning as in claim 1 and $O^B$ and $A^B$ represent OH, the A group, respectively, bearing hydroxy-blocking groups where appropriate, and $Z^B$ represents a Z group bearing an amino-blocking group where appropriate,

(a) with an acid to remove any hydroxy-protecting groups and

(b) with a base to remove any amino-protecting group.

4. Use of a compound of claim 1 for the preparation of a pharmaceutical composition.

5. The use in the manufacture of a medicament, for treating a patient suffering from a neoplastic disease, of a compound of claim 1.

6. Use according to claim 5 wherein a neoplastic disease is a leukemia.

7. Use according to claim 5 wherein the neoplastic disease is a melanoma.

8. Use according to claim 5 wherein the neoplastic disease is a carcinoma.

9. Use according to claim 5 wherein the neoplastic disease is a sarcoma.

10. Use according to claim 5 wherein the neoplastic disease is a mixed type of neoplasia.

11. Use of a compound of claim 1 for the preparation of a pharmaceutical composition useful for inhibiting a purine nucleoside phosphoylase in a neoplasm of a patient suffering from a neoplastic disease which is dependent on purine nucleoside phosphoylase for its growth.

12. A pharmaceutical composition comprising a therapeutically effective amount of a compound of claim 1 in admixture with one or more pharmaceutically acceptable carriers or excipients.

13. A pharmaceutical composition according to claim 12 for treating a patient suffering from a neoplastic disease.

Claims for the following Contracting State: ES

1. A process for preparing a compound of the formula 3

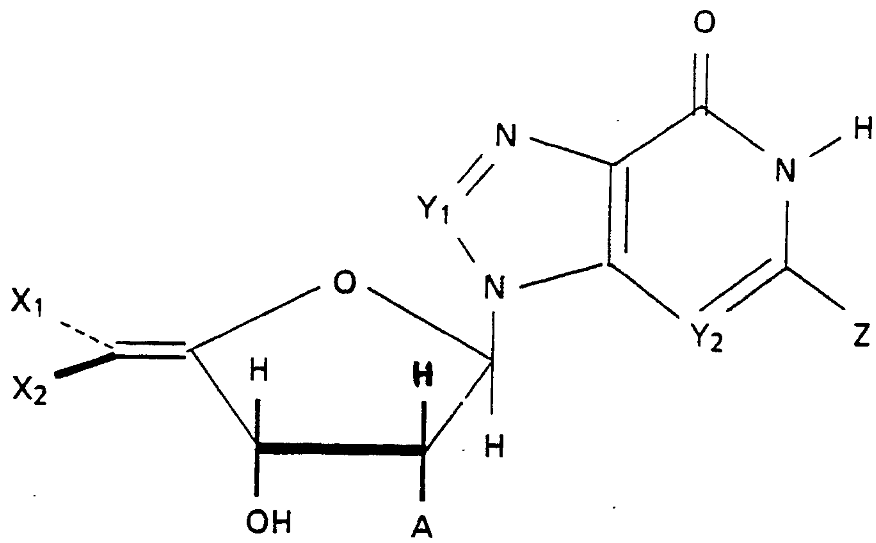

(3)

wherein

$X_1$ and $X_2$ are each independently hydrogen or halogen with the proviso that at least one of $X_1$ and $X_2$ is a halogen atom,

A is hydrogen or hydroxy

$Y_1$ is nitrogen, a CH group, a CCl group, a CBr group or a $CNH_2$ group,

$Y_2$ is nitrogen or a CH group, and

Z is hydrogen or halogen

comprising reacting an acidic oxidative agent with a compound of the formula 2

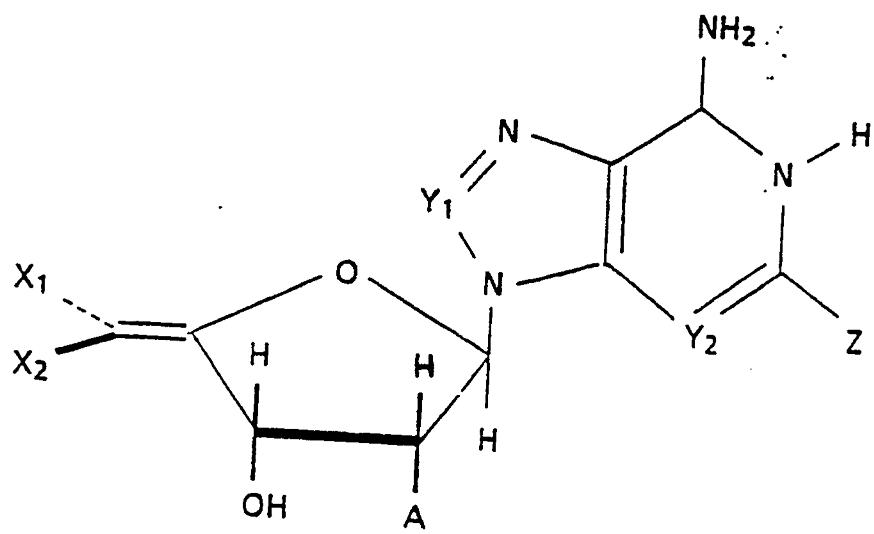

(2)

wherein the substituents $X_1$, $X_2$, A, $Y_1$ and $Y_2$ and Z have the same meaning as above.

2. A process for preparing a compound of the formula 1

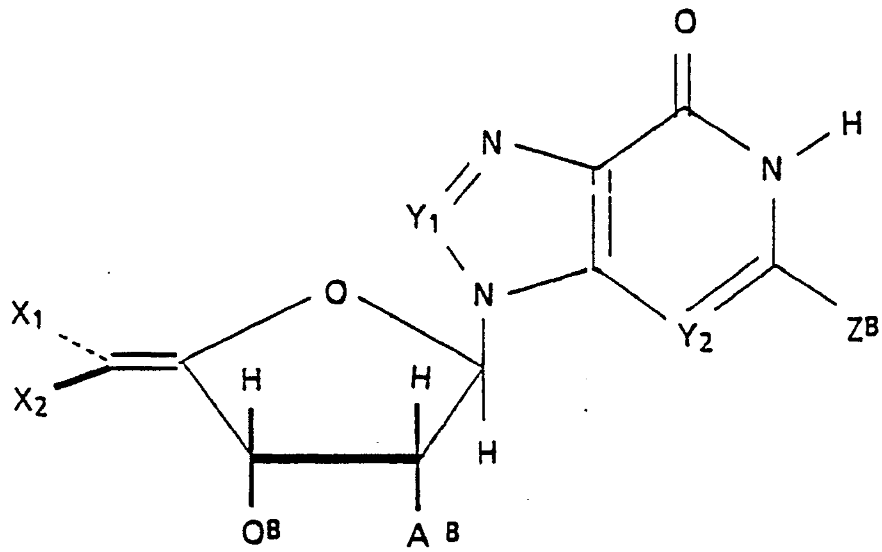

wherein $X_1$, $X_2$, $Y_1$ and $Y_2$ have the same meaning as in claim 1 and $O^B$ and $A^B$ represent OH, the A group, respectively, bearing hydroxy-blocking groups where appropriate, and $Z^B$ represents a Z group bearing an amino-blocking group where appropriate,

(a) with an acid to remove any hydroxy-protecting groups and

(b) with a base to remove any amino-protecting group.

4. Use of a compound of claim 1 for the preparation of a pharmaceutical composition.

5. The use in the manufacture of a medicament, for treating a patient suffering from a neoplastic disease, of a compound of claim 1.

6. Use according to claim 5 wherein a neoplastic disease is a leukemia.

7. Use according to claim 5 wherein the neoplastic disease is a melanoma.

8. Use according to claim 5 wherein the neoplastic disease is a carcinoma.

9. Use according to claim 5 wherein the neoplastic disease is a sarcoma.

10. Use according to claim 5 wherein the neoplastic disease is a mixed type of neoplasia.

11. Use of a compound of claim 1 for the preparation of a pharmaceutical composition useful for inhibiting a purine nucleoside phosphoylase in a neoplasm of a patient suffering from a neoplastic disease which is dependent on purine nucleoside phosphoylase for its growth.

European Patent Office

# EUROPEAN SEARCH REPORT

Application Number

EP 90 11 9494

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 334 361 (MERRELL DOW PHARMACEUTICALS INC.)<br>* Abstract; pages 2,3 * | 1,4-13 | C 07 H 19/167<br>C 07 H 19/173<br>C 07 H 19/052<br>C 07 H 19/056<br>A 61 K 31/70 |
| Y | EP-A-0 304 889 (MERRELL DOW PHARMACEUTICALS INC.)<br>* Abstract; page 2, line 1 - page 3, line 40 * | 1,4-13 | |
| Y | EP-A-0 045 944 (YAMASA SHOYU K.K.)<br>* Abstract * | 1,4-13 | |
| A | THE JOURNAL OF ORGANIC CHEMISTRY, vol. 44, no. 3, 2nd February 1979, pages 400-406, American Chemical Society; S.D. DIMITRIJEVICH et al.: "Halo sugar nucleosides 6. Synthesis of some 5'-deoxy-5'-iodo and 4',5'-unsaturated purine nucleosides"<br>* Abstract; page 401, compound 8; page 402, compound 22 * | 1 | |
| Y | EP-A-0 329 348 (ELI LILLY & CO.)<br>* Abstract * | 1,4-13 | TECHNICAL FIELDS SEARCHED (Int. Cl.5) |
| Y | EP-A-0 198 387 (KANEGAFUCHI CHEMICAL INDUSTRY CO., LTD)<br>* Abstract * | 1,4-13 | C 07 H 19/00<br>A 61 K 31/00 |

The present search report has been drawn up for all claims

| Place of search | Date of completion of search | Examiner |
|---|---|---|
| The Hague | 25 January 91 | SCOTT J.R.M. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document